# EUROPEAN PATENT APPLICATION

(11) **EP 1 741 394 A1**
(43) Date of publication of application: **10.01.2007**
(21) Application number: 05076559.3
(22) Date of filing: 08.07.2005
(51) Int. Cl.: A61B 17/16, A61B 18/22

(54) **Surgical drill system and surgical drill bit to be used therein**

(71) Applicant: Technische Universiteit Delft, 2628 BL Delft (NL)
(72) Inventor: Margallo Balbas, Eduardo, 2624 DN Delft (NL); Wieringa, Peter A., 2628 CD Delft (NL); French, Patrick James, 2625 KS Delft (NL); Lee, Ruben Armstrong, 2774 PC Voorburg (NL); Breedveld, Paulus, 2806 DK Gouda (NL)
(74) Representative: Van Breda, Jacobus

(57) **Abstract**

Surgical drill system comprising a drill bit and means for imaging the vicinity of the drill bit tip, saId means comprising:
at least one optical fiber having a distal end and a proximal end,
said distal end being located adjacent said drill bit tip,
an optical processing unit,
said proximal end of said at least one optical fiber being operatively connected to said processing unit,
said at least one optical fiber directing light transmitted therethrough to the vicinity of said drill bit tip and collecting light reflected back from the vicinity of the drill bit tip,
whereby an image in the vicinity of the drill bit tip is produced, wherein at least one optical fiber is housed within the drill bit.

## Description

The invention relates to a surgical drill system and to a surgical drill bit to be used therein.

Surgical drills are used to make holes in bone necessary for placement of various types of implants. They are used in orthopaedics but the use has also expanded to dentistry. In the following the invention will be elucidated with reference to dental applications although the invention is not limited to this specific field.

In 2004 the dental reconstructive implant market is estimated at approximately $1.145 million, growing 14-16% annually. The potential for this treatment is still large, as 69% of adults from 35 to 44 years were missing one or more teeth in 2000. An increasingly old population may thus benefit from a prosthetic replacement technique.

Furthermore, the clinical interest of reconstructive treatment should not be ignored. An approxiate number of 910.000 osseointegrated implants were placed in the year 2000 only in the USA. This large figure implies that surgical complications affect many patients, even if their relative incidence is not high. Life-threatening perioperative bleeding, nerve lesions, damage to adjacent teeth and inadvertent perforation of the sinus membrane are reported persisting pitfalls.

The surgical procedures start with appropriate anaesthesia of the operating area. Then soft tissue is displaced by carefully cutting and detaching the periosteum membrane from the bone. This process is called flap reflection.

Once the bone is exposed, the first stage is completed and the instrument-set is changed. A series of rotating drill bits are brought into the operating area. During their usage, sufficient saline irrigation should be present, in order to keep the drill cool throughout the procedure. It has to be mentioned that some drilling systems incorporate internal irrigation through channels in the drill. Frequently, small bone fragments have to be retired from the drill end and a vertical oscillating movement is recommended for the saline solution to reach the bottom.

Despite all ongoing efforts to enhance patient safety, nerve damage and bleeding due to vessel injuries during bone drilling are still worrying complications in dental implant placement. Other surgical problems that trouble the practitioner are, inadvertent perforation of the sinus membrane, leading to sinusitis and oroantral communication, and damage to adjacent teeth.

Injury to the local nerve induces transient or permanent paresthesia (sensory disturbance) in the area surrounding the chin, in part of the tongue and in the gingiva.

Damage to main vessels in the mouth floor is considered a rare complication of implant placement. However, at least ten of these potentially life-threatening episodes can be found in nine published articles. In them, damage to a branch of the lingual or facial arteries, mostly with the surgical drill, leads to a profuse haemorrhage. Blood streaming into the neighbouring tissues produces rapid swelling and tongue elevation, compressing and obstructing the airways. All reported cases had to be attended in a hospital and most of them needed recovery in the intensive care unit.

From an anatomical point of view, the sub-mental and sub-lingual arteries are the two critical vessels in this kind of surgery. Anatomical variability makes their position and size somewhat uncertain and anastomoses between both of them are known to be common, what complicates haemorrhage contention. Complications are especially probable if the vessels run close to the jaw, for example along an extended *fossa lingualis.* The soft tissue anatomy that is relevant to assess risk appropriately in a preoperative phase is normally not available in a panoramic radiography or in a CT scan.

US-B-6,419,484 teaches a combination of a dental drill and means for imaging the vicinity of the drill tip, said means comprising at least one optical fiber having a distal end and a proximal end, said distal end being located adjacent said drill tip, and further an optical coherence domain reflectometry (OCDR) unit, whereby the proximal end of said at least one optical fiber is operatively connected to said OCDR unit, and said at least one optical fiber directing light transmitted therethrough to the vicinity of said drill tip and collecting light reflected back from the vicinity of the drill tip, whereby an image of the vicinity of the drill tip is produced.

The optical coherence domain reflectometry system connected via the fiber optics to the dental drill according to this citation enables imaging of an area in front of the drilling area or ablated surface. This allows the user to identify the boundary between decayed and normal enamel (or dentine) or the boundary adjacent sensitive tissue or the nearness of nerves etc. Accordingly the drill of US-B-6,419,484 is surrounded with one or more optical fibers connected to the optical coherence domain reflectometry system to image several millimetres ahead of the ablation surface.

The instant invention is aimed at improving the reliability and accuracy of this known system.

The surgical drill system according to the invention is to this end characterized in that said at least one optical fiber is housed within the drill bit.

With this measure it is possible that the investigative light is brought into close proximity of the bone which is being drilled, allowing for a more reliable and early detection of dangerous areas where drilling has to be avoided.

One possible and relatively straightforward means of housing the at least one optical fiber is to place same in a channel along the axis of the drill bit.

In a further preferred embodiment the surgical drill system of the invention is characterized in that there is a plurality of optical fibers housed in the drill bit that are arranged around the axis of the drill bit.

The thus during operation of the drill bit rotating optical fibers allow for a better detection of dangerous areas at the circumference of the rotating drill bit, albeit at the expense of an analysis that is required of the light reflected back from the vicinity of the drill bit tip that keeps track of the rotating angle of the drill bit, during transmission of the light.

To facilitate the surgical drill system to operate appropriately it is preferable that the at least one optical fiber is operatively connected to said optical processing unit through a rotatable optical interface.

For the rotatable optical interface use can be made of a micro-optical rotary joint as is taught by M. Stark, M. Rank, M. Schmidt, G. Popp, and H. Poisel in "Micro-optical rotary joint for multichannel communication via a rotating surface" incorporated in G.S. Mecherle, editor, Free-Space Laser Communication Technologies XVII, volume 5710, pages 240-50. SPIE, Apr 2005.

To restrict the accuracy requirements on the construction of the surgical drill system of the invention it is preferred that the said at least one optical fiber is at its distal end provided with a micro-lens.

Several types of micro lenses could be used for this purpose but it is preferred that the micro-lens is selected from the group comprising ball-lens, C-lens, grin-lens.

The invention is also embodied in a surgical drill bit, which can be used in the surgical drill system as elucidated above. This surgical drill bit is according to the invention characterized by at least one optical fiber housed therein.

Further preferable embodiments of the surgical drill bit of the invention are specified in claims 8-11.

The invention will hereinafter further be elucidated with reference to a preferred embodiment of the surgical drill system and surgical drill bit of the invention and with reference to the drawing.

In the drawing:
Fig. 1 schematically shows the surgical drill system of the invention, and the light distribution in a model of the mandibular canal; and
Fig. 2 represents the time response of reflected photons, according to the same model.

US-B-6,419,484 represents an optical coherence domain reflectometry guided dental drill system of the prior art which is further improved by the instant invention. The general working principles of the system of the invention are disclosed in US-B-6,419,484 which document is incorporated herein by reference.

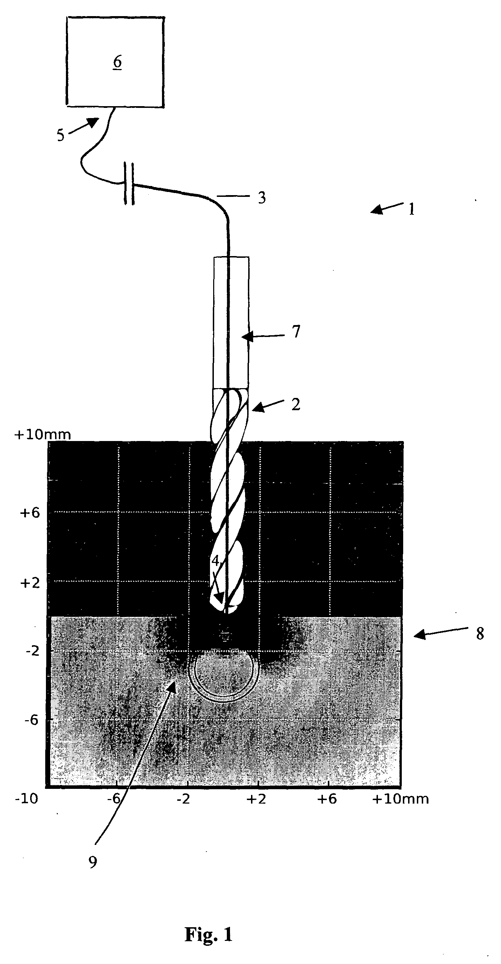

Fig. 1 schematically shows the surgical drill system 1 of the invention comprising a drill bit 2 and means for imaging the vicinity of the drill bit tip.

The said means for imaging the vicinity of the drill bit tip comprise in the shown example one optical fibre 3 having a distal end 4 located adjacent the drill bit tip and a proximal end 5 which is connected to an optical processing unit 6.

Through the optical fibre 3 light is transmitted to the vicinity of the tip of the drill bit 2 and at said distal end 4 light is collected that is reflected back from said vicinity of the tip of the drill bit 2. Using said light that is reflected back, the processing unit 6 images the vicinity of the drill bit tip.

As Fig. 1 shows, the optical fibre 3 is at least partly housed within the drill bit 2 in a channel 7 of this drill bit 2.

Another possibility is to house a plurality of optical fibres in the drill bit 2 and arrange them around the axis of the drill bit 2. This is not shown in the drawings but completely clear for the person skilled in the art so that a further elucidation thereof can be dispensed with.

It is preferable that the optical fibre 3 is operatively connected to the processing unit 6 through a rotatable optical interface such as the micro-optical rotary joint mentioned above.

In a manner known to the person skilled in the art the optical fiber is at its distal end 4 provided with a micro lens such as one selected from the group comprising ball lens, C-lens and GRIN lens.

In Fig. 1, also the distribution of light-absorption is shown schematically. It can be seen that the superimposed canal influences heavily the light distribution in tissue 8, with significant light fluence in the area of interest 9. This light distribution is to be studied and to this end use can be made of different techniques.

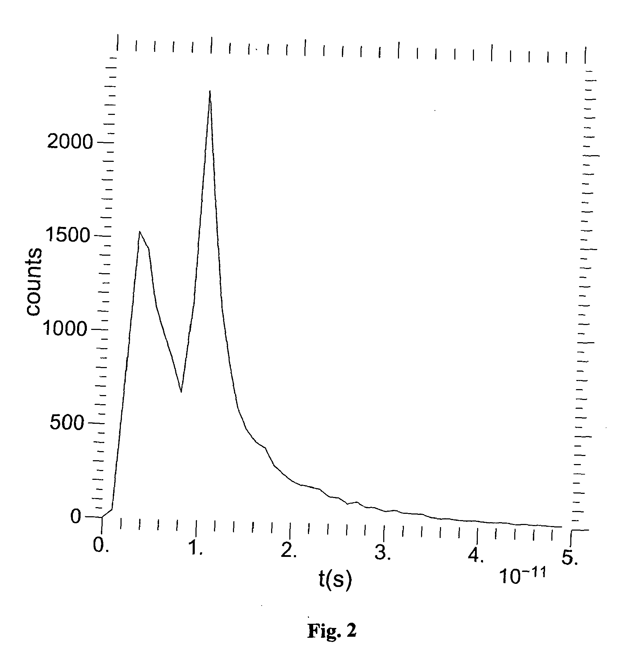

A first technique is represented by the time resolved reflected signal that is shown in Fig. 2. The reflected signal exhibits two peaks, the second peak being due to heavy scattering in the cortical enclosure of the canal in the bone structure. It is also possible to apply spatial or frequency domain methods. It is preferred however to apply optical coherence tomography as is generally described by J.G. Fujimoto and M.E. Brezinski in Biomedical Photonics Handbook, chapter 13. CRC Press, Boca Raton, 2003. ISBN 0-8493-1116-0.

## Claims

1. Surgical drill system (1) comprising a drill bit (2) and means for imaging the vicinity of the drill bit tip, said means comprising:
at least one optical fiber (3) having a distal end (4) and a proximal end (5),
said distal end (4) being located adjacent said drill bit tip,
an optical processing unit (6),
said proximal end (5) of said at least one optical fiber (3) being operatively connected to said processing unit (6),
said at least one optical fiber directing light transmitted therethrough to the vicinity of said drill bit tip and collecting light reflected back from the vicinity of the drill bit tip,
whereby an image in the vicinity of the drill bit tip is produced, **characterized in that** said at least one optical fiber (3) is housed within the drill bit (2).

2. Surgical drill system according to claim 1, **characterized in that** said at least one optical fiber (3) is housed in a channel (7) along the axis of the drill bit (2).

3. Surgical drill system according to claim 1, **characterized in that** there is a plurality of optical fibers housed in the drill bit (2) that are arranged around the axis of the drill bit.

4. Surgical drill system according to anyone of the claims 1-3, **characterized in that** the at least one optical fiber (3) is operatively connected to said optical processing unit (6) through a rotatable optical interface.

5. Surgical drill system according to anyone of the preceding claims, **characterized in that** the said at least one optical fiber (3) is at its distal end (4) provided with a micro-lens.

6. Surgical drill system according to claim 5, **characterized in that** the micro-lens is selected from the group comprising ball-lens, C-lens, grin-lens.

7. Surgical drill bit (2) **characterized by** at least one optical fiber (3) housed therein.

8. Surgical drill bit (2) according to claim 7, having an axis, **characterized in that** the at least one optical fiber (3) is housed in a channel (7) along said axis.

9. Surgical drill bit according to claim 7, **characterized in that** there is a plurality of optical fibers housed therein that are arranged around the said axis.

10. Surgical drill bit according to anyone of the claims 7-9, **characterized in that** the said at least one optical fiber (3) is at its distal end provided with a micro-lens.

11. Surgical drill bit according to claim 10, **characterized in that** the micro-lens is selected from the group comprising ball-lens, C-lens, grin-lens.
